Europäisches Patentamt

European Patent Office

Office européen des brevets

(11) Publication number: **0 204 583 B1**

# EUROPEAN PATENT SPECIFICATION

(45) Date of publication of patent specification: **25.03.92** (51) Int. Cl.⁵: **G01N 33/02**, G01N 33/48

(21) Application number: **86304344.4**

(22) Date of filing: **06.06.86**

The file contains technical information submitted after the application was filed and not included in this specification

(54) **A method of preparing a food sample for testing for human food sensitivity.**

(30) Priority: **06.06.85 GB 8514359**

(43) Date of publication of application:
**10.12.86 Bulletin 86/50**

(45) Publication of the grant of the patent:
**25.03.92 Bulletin 92/13**

(84) Designated Contracting States:
**AT BE CH DE FR GB IT LI LU NL SE**

(56) References cited:
**EP-A- 0 149 383
CH-A- 629 255
US-A- 4 267 275**

**PATENT ABSTRACTS OF JAPAN, unexamined applications, field C, vol. 8, no. 19, 26 January 1984, The Patent Office Japanese Government; p. 142C207**

**LEUCOCYTE CYCOTOXIC TESTING, report presented at Annual Meeting of Otolaryncig Allergy, 13-15 September 1984; A.WOJDANI et al., pp. 67-91**

(73) Proprietor: **Larkhall Laboratories plc
225-229 Putney Bridge Road
London SW15 2PY(GB)**

(72) Inventor: **Blau, Cyril Aaron
19 Boscombe Road Merton Park
London, SW19 3AX(GB)**
Inventor: **Lawrence, Keith Ramsay
26 Marius Road Balham
London, SW17 7OO(GB)**

(74) Representative: **West, Alan Harry et al
R.G.C. Jenkins & Co. 26 Caxton Street
London SW1H 0RJ(GB)**

## Description

The present invention is concerned with a method of preparing a test plate containing a water-soluble protein fraction derived from a food suitable for use in a test for human sensitivity to the food, and more particularly to a method of preparing such a protein test plate which avoids the breakdown of the water soluble proteins which are representative of the actual food used in the test.

Up until the present time, it has been found difficult and/or time consuming to identify accurately food sensitivities in individuals.

A known method by which these may be identified is by subjecting an individual to a so-called exclusion diet. This involves initially placing the individual on a very simple diet such as lamb, pears and distilled water for a period of about 5 days. Subsequently, individual foods are successively reintroduced into the diet in periods of about 5 days and the response to each food by the individual is monitored to detect any reaction.

A more recent method of detecting food sensitivities involves the use of a cytotoxic test, which is a method of rapidly identifying such sensitivities by laboratory testing. This test is based on alterations in the appearance and motility of granulocytes when added to the various water-soluble protein fractions. It is not yet certain why alterations do occur though it is noted that granulocytes form an important part of the human defence mechanism.

To confirm the results of such a test, the above mentioned exclusion diet is still recommended. However, the diet may be restricted to include only the foods to which the individual has been found previously sensitive. The introduction of cytotoxic testing in conjunction with an exclusion diet therefore identifies food sensitivities more easily and rapidly than previously possible.

The cytotoxic test itself is relatively simple, but requires a high standard of laboratory expertise in interpreting the observed morphological degeneration of granulocytes which takes place in the presence of water-soluble protein fractions derived from a food. This requires a subjective evaluation of the degeneration process by a qualified technician and has provided a basis for criticism of cytotoxic tests. However, several studies have produced results from such testing having a high degree of reproducability when carried out by experienced technicians.

Because the cytotoxic test is extremely sensitive it is necessary to ensure that the water-soluble protein fractions suffer no contamination or decomposition during their preparation. Up until the present time, a great many of the problems associated with cytotoxic testing have been due to both the infection and the breakdown of the water-soluble protein fraction during the drying process. The present invention provides both a quick and contamination-free method of producing water-soluble protein fractions suitable for cytotoxic testing, thus improving the overall reliability of such a test.

According to one aspect of the present invention there is provided a method of preparing a test plate containing a water-soluble protein fraction derived from a food and suitable for testing for human sensitivity to the food comprising the steps of:

preparing an aqueous solution of the water-soluble protein fraction from a food/water dispersion by sterile filtration;

depositing the aqueous solution of the water-soluble fraction in a well formed in a test plate; and

freeze drying the aqueous solution to deposit the food sample in the well.

According to a further aspect of the present invention, there is provided a plate for use in a test for human sensitivity to a plurality of different foods comprising a plurality of wells each having deposited therein a water-soluble protein fraction derived from a different food obtainable the method described above.

Finally, the present invention provides, as a further aspect a method of in vitro testing for human sensitivity to various foods comprising the step of examining human granulocytes added to the plurality of different water-soluble protein fractions deposited in the wells of the above plate.

The present invention therefore provides a method of preparing water-soluble protein fractions derived from selected foods which may be used in a test for human sensitivity to the food. In practice, a suitable test plate includes a plurality of wells, which preferably number between 40 and 60, and more preferably 48, in which a similar plurality of water-soluble protein fractions are individually placed. The water-soluble protein fractions of the foods may be prepared by first dispersing the selected food in purified water, separating the water-soluble and insoluble fractions and then freeze-drying the aqueous fraction, thus depositing the protein fraction in each well.

The aqueous fraction deposited in the well may be frozen prior to the step of freeze-drying.

An embodiment of the invention will now be described.

## Preparation of the Water-Soluble Protein Fraction of the Food

The food to be tested is firstly thoroughly dried. Specially prepared dried foods may be purchased from specialist suppliers such as Hollister-Steir Laboratories and Crystal Laboratories.

A portion of the food is dispersed in preferably from 150 to 1500 times and more preferably in about 500 times its own weight of purified water. The process of dispersion is carried out at room temperature and with agitation of the mixture for a period of preferably between 6 to 12 hours and more preferably about 8 hours.

The insoluble fraction of the food, is filtered off using a known type of filter paper capable of trapping bacteria. This filter paper has a pore size of less than 2 $\mu$m. The insoluble fraction of the food which is filtered off includes any bacteria or yeasts which may have contaminated the food.

As only a minute fraction of the food is required for the actual sensitivity test, one advantage of using the water-soluble protein fraction of the food for testing is the ease by which very dilute solutions of this fraction can be prepared. The water used in the preparation of the water-soluble protein fraction is purified to prevent any contamination with bacteria, yeasts or other substances. This water is prepared by firstly passing it through an ion exchange resin to remove any chemical impurities, and secondly, if necessary, by filtering it through a fine pored filter paper to remove bacteria and yeasts.

## Preparation of the Test Plate

Once the aqueous solution of the water-soluble protein fraction has been prepared, then a small amount of this fraction preferably between 20 and 40 microlitres and more preferably about 30 microlitres, is added to a well formed in the test plate. The test plate preferably comprises a multiplicity e.g. 48 of such wells, and aqueous solutions of protein fractions prepared from an equal plurality of different foods are added to these wells.

It is preferred that such additions are carried out by way of an automated process. An example of such a process is to control an auto-sampler having a single auto-pipette mounted on it with a computer to carry out the following sequence of steps:

(i) fill the auto-pipette with a first predetermined amount of an aqueous solution of the water-soluble protein fraction prepared from a first food, the first predetermined amount being sufficient to deposit a second predetermined amount of the solution into a well of each of a plurality of pre-arranged test plates;

(ii) deposit the second predetermined amount of the solution from the auto-pipette into each of the first wells of the plurality of pre-arranged test-plates;

(iii) clean the auto-pipette; and

(iv) fill the auto-pipette with a first predetermined amount of an aqueous solution of the water-soluble protein fraction prepared from a second food...etc.

A further way of automating the addition of the aqueous solutions to the wells is by prearranging a plurality of auto-pipettes to have substantially the same orientation as the plurality of wells of a test plate. In use, the pipettes are simultaneously filled with the different solutions and then positioned over the test plate which is located at a predetermined position. The solutions are then simultaneously added to the appropriate wells. The test plate is then removed e.g. by conveyor, and replaced by a fresh one.

The test plate is then preferably frozen in a freezer. This step prevents contamination of the solutions by bacteria and yeasts and also improves the efficiency of the freeze-drying process.

The frozen solutions are then freeze-dried for a period sufficient to evaporate substantially all the water in which the fraction is dissolved. The freeze-drying step minimises the risk of contamination of the protein fractions during the process. A further advantage of this process is that the reduced temperature avoids any risk of thermal decomposition of the protein.

Preferably freeze-drying is carried out for a period of between 1 to 10 hours and more preferably 4 to 8 hours. The plate is preferably freeze-dried at a temperature of between -40° to -70°C and more preferably between -50° and -60°C. The pressure used during the freeze-drying process is preferably between $1 \times 10^{-4}$ and $1 \times 10^{-3}$ millibars and more preferably about $2.5 \times 10^{-4}$ millibars.

After the freeze-drying process has been completed, only the purified water-soluble protein fractions (representative of the food under test) remain in the wells of the test plate.

Previously, in the preparation of such test plates, the water has been evaporated in the open at or above ambient temperature resulting in possible thermal decomposition of the proteins and/or environmental contamination of them by airborne yeasts or bacteria. Thus protein fractions prepared in this way are generally unrepresentative of the initial foods, and give erroneous results.

### The Prepared Plates

After the freeze-drying process, the test plates may be immediately used in a cytotoxic test, or may be stored by individually wrapping them in clingfilm, and then maintaining them at a temperature of about -10°C until required for use. It is believed that the plates are capable of being stored for at least a year without deterioration.

### The Test Plates

The plates used in the present invention preferably comprise a transparent plastic material, having moulded therein a plurality of wells. It is preferred that the plastic material is a homopolymer e.g. PVC or polystyrene as such polymers generally have no additives in them which might affect the sensitive cytotoxic test. The material from which the plates are formed is preferably between 0.25 and 2.0, and more preferably about 0.5 millimetres in thickness.

An example of the preparation of a test plate will now be described.

### EXAMPLE 1

10 milligrammes of 48 different dried foods (obtained from Hollister-Steir Laboratories and Crystal Laboratories), as set out in any one of Tables 1, 2 or 3 of the Appendix, were each dispersed at room temperature with agitation in 5 millilitres of purified water. The solutions were then filtered, and the water-soluble protein fraction isolated.

30 microlitres of each of the 48 fractions were then added to separate wells formed in a cytotoxic test plate comprising PVC. The plate was then placed in a freezer, to freeze the solution.

The plate was then put in an Edwards EF4 Modulyo freeze-dryer for six hours at a temperature of about -55°C and at a pressure of about $2.5 \times 10^{-4}$ millibars. After the freeze-drying process was completed, the test plate was wrapped in clingfilm and stored at -10°C for six months. The plate was then used in a cytotoxic test described below.

### The Cytotoxic Test

The cytotoxic test is a rapid method of identifying food, chemical and inherent sensitivities by in vitro testing. A method of carrying out such a test is described in a paper presented by Aristo Wojdani, Elizabeth Stein, and Geoffrey Cheung at the annual meeting of the American Academy of Otolaryngicallergy on September 13-15, 1984 Las Vegas, Nevada.

The preferred cytotoxic tests to be used in connection with the plates of the present invention uses fresh blood taken from a patient who has been deprived of all types of food for at least twelve hours. A sufficient quantity of blood is taken (generally about 5 ml) for each batch of 48 foods to be tested. A full cytotoxic test covers 144 foods protein fractions of which have been deposited on 3 plates each containing 48 protein fractions.

0.5 millilitres of 3.85% trisodium citrate per 5 ml of blood are then added to the blood, and the mixture subjected to centrifugation at 1000 revolutions per minute for 30 minutes. This process separates the mixture into three fractions, a bottom layer comprising red blood cells, a buffy layer containing white blood cells, and lastly a top layer containing plasma. The plasma and buffy layers are extracted using a long pasteur pipette, and 30 microlitres of this extract are added to each of the 48 protein fractions on the plate. The plate is then incubated for 10 minutes at 36.9°C, which corresponds to body temperature. The plates are then removed and allowed to stand for between 10 to 30 minutes.

Each well of the plate is then viewed at X40 magnification under a microscope, and is examined for any abnormalities in the white cells or platelets.

If no abnormalities are found then this is interpreted to be a negative reaction, and so the patient is classified as not being sensitive to the particular food found in the well. If there are abnormalities, such as lysis or removal of the cell membrane, then an allergic reaction has been shown by the patient to the food, and the strength of reaction is graded according to the range and degree of abnormalities. The system of grading currently being used is illustrated in Table 4.

4

TABLE 4

| % Damage to Granulocytes | Response Grade |
|---|---|
| 0-24 | 0 |
| 25-44 | 1 |
| 45-64 | 2 |
| 65-84 | 3 |
| 85-100 | 4 |

Where red blood cells are also extracted from the centrifuged solution, then abnormalities may also be found in these in the case of a grade 4 response.

The buffy layer also contains platelets found in the blood which assist in the clotting process, and in a positive response, the platelets can be found to aggregate.

## APPENDIX

### Table 1

| | | |
|---|---|---|
| 1. Cane Sugar | 2. Beet Sugar | 3. Chocolate |
| 4. Coffee | 5. Cola | 6. Malt |
| 7. Saccharine | 8. Tea | 9. Butter |
| 10. Cheese | 11. Egg Yolk | 12. Egg White |
| 13. Milk-Evaporated | 14. Milk-Goats | 15. Milk-Whole |
| 16. Cod | 17. Haddock | 18. Mackerel |
| 19. Herring | 20. Apple | 21. Pilchard |
| 22. Grapefruit | 23. Lemon | 24. Orange |
| 25. Peanut | 26. Pear | 27. Barley |
| 28. Corn | 29. Rice | 30. Rye |
| 31. Oats | 32. Wheat | 33. Beef |
| 34. Chicken | 35. Lamb | 36. Pork |
| 37. Turkey | 38. Haricot Beans | 39. Cabbage |
| 40. Carrot | 41. Onion | 42. Pea |
| 43. Potato | 44. Soya | 45. Tomatoes |
| 46. Peppermint | 47. Yeast Bakers | 48. Yeast Brewers |

## Table 2

| 49. Honey | 50. Roquefort cheese | 51. Cottage Cheese |
|---|---|---|
| 52. Yoghurt | 53. Avacado | 54. Hazel |
| 55. Almond | 56. Apricot | 57. Banana |
| 58. Brazil Nut | 59. Cashewnut | 60. Cherry |
| 61. Chestnut | 62. Coconut | 63. Grape |
| 64. Lime | 65. Peach | 66. Pineapple |
| 67. Plum | 68. Strawberry | 69. Walnut |
| 70. Rhubarb | 71. Aubergine | 72. Buckwheat |
| 73. Millet | 74. Brussels Sprout | 75. Cauliflower |
| 76. Celery | 77. Garlic | 78. Lentil |
| 79. Lettuce | 80. Mushroom | 81. Mustard |
| 82. Radish | 83. Spinach | 84. Spring Bean |
| 85. Turnip | 86. Brocolli | 87. Greenpepper |
| 88. Beetroot | 89. Mint | 90. Black Pepper |
| 91. Tapioca | 92. Sunflower | 93. E.102 |
| 94. E.120 | 95. E.123 | 96. E.142 |

## Table 3

| | | |
|---|---|---|
| 97. Carob | 98. Anchovy | 99. Coley |
| 100. Flounder | 101. Halibut | 102. Oyster |
| 103. Shrimp | 104. Tuna | 105. White Bait |
| 106. Crab | 107. Sole | 108. Hake |
| 109. Sardine | 110. Scampi | 111. Prawns |
| 112. Rainbow Trout | 113. Blackberry | 114. Cantaloupe |
| 115. Date | 116. Fig | 117. Gooseberry |
| 118. Mango | 119. Olive | 120. Watermelon |
| 121. Hops | 122. Sago | 123. Asparagus |
| 124. Artichoke | 125. Duke | 126. Rabbit |
| 127. Marrow | 128. Parsnip | 129. Cucumber |
| 130. Bay | 131. Chilli | 132. Cinnamon |
| 133. Clove | 134. Ginger | 135. Marjoram |
| 136. Nutmeg | 137. Oregano | 138. Paprika |
| 139. Parsley | 140. Rosemary | 141. Tarragon |
| 142. Thyme | 143. Turmeric | 144. Monosodium Glutamate |

**Claims**

1. A method of preparing a test plate containing a water-soluble protein fraction derived from a food and suitable for testing for human sensitivity to the food, comprising the steps of:

preparing an aqueous solution of water-soluble protein fraction from a food/water dispersion by sterile filtration;

depositing the aqueous solution of the water-soluble fraction in a well formed in a test plate; and

freeze-drying the aqueous solution to deposit the fraction in the well.

2. A method according to claim 1, wherein the food is dispersed in from 150 to 1500 times its own weight of purified water.

3. A method according to claim 1 or claim 2, wherein the aqueous solution of the fraction is deposited in an automated process.

4. A method according to any preceding claim, wherein from 20 to 40 $\mu$l of the aqueous solution is deposited in the well.

5. A method according to any preceding claim, wherein the deposited aqueous solution in the well is frozen prior to the freeze-drying step.

6. A method according to any preceding claim, wherein freeze-drying is carried out for a period of from 1 to 10 hours.

7. A method according to any proceding claim, wherein freeze-drying is carried out at a temperature of from -40° to -70°C.

8. A method according to any proceding claim, wherein freeze-drying is carried out under a pressure of from $1 \times 10^{-4}$ to $1 \times 10^{-3}$ millibars.

9. A plate for use in a test for human sensitivity to a plurality of different foods comprising a plurality of wells each having deposited therein a water-soluble protein fraction derived from a different food each obtainable according by the method of any of claims 1 to 8.

10. A method of in vitro testing for human sensitivity to various foods comprising the step of examining human granulocytes added to the plurality of different water-soluble protein fractions deposited in the wells of a plate according to claim 9.

**Revendications**

1. Méthode pour préparer une plaque de test contenant une fraction protéinique soluble dans l'eau dérivée d'un aliment, et adaptée pour tester la sensibilité humaine à la nourriture, comprenant les étapes consistant à :

   préparer une solution aqueuse de la fraction protéique soluble dans l'eau, à partir d' aliment dispersé dans l'eau, par filtration stérile ;

   déposer la solution aqueuse de la fraction soluble dans l'eau dans un puits formé dans une plaque de test ; et

   lyophiliser la solution aqueuse pour déposer la fraction dans le puits.

2. Méthode selon la revendication 1, dans laquelle la nourriture est dispersée dans de 150 à 1500 fois son propre poids d'eau purifiée.

3. Méthode selon la revendication 1 ou la revendication 2, dans laquelle la solution aqueuse de la fraction est déposée par un procédé automatique.

4. Méthode selon l'une quelconque des revendications précédentes, dans laquelle de 20 à 40 $\mu$litres de la solution aqueuse sont déposés dans le puits.

5. Méthode selon l'une quelconque des revendications précédentes, dans laquelle la solution aqueuse déposée dans le puits, est congelée préalablement à l'étape de lyophilisation.

6. Méthode selon l'une quelconque des revendications, dans laquelle la lyophilisation est effectuée pendant une période allant de 1 à 10 heures.

7. Méthode selon l'une quelconque des revendications précédentes, dans laquelle la lyophilisation est effectuée à une température allant de -40° à -70°C.

8. Méthode selon l'une quelconque des revendications précédentes, dans laquelle la lyophilisation est effectuée à une pression de $1 \times 10^{-4}$ à $1 \times 10^{-3}$ millibars.

9. Plaque pour emploi dans un test de la sensibilité humaine à une pluralité de nourritures différentes, comprenant une pluralité de puits, chacun ayant au fond une fraction protéinique soluble dans l'eau, dérivée d'un aliment différent, pouvant chacun être obtenue selon la méthode de l'une quelconque des revendications de 1 à 8.

10. Méthode pour tester in vitro la sensibilité humaine à des nourritures variées comprenant l'étape consistant à examiner les granulocytes humains ajoutés à la pluralité de différentes fractions protéini-

8

ques solubles dans l'eau, déposées dans les puits d'une plaque selon la revendication 9.

**Patentansprüche**

1. Verfahren zur Bereitung einer Probenplatte, die eine wasserlösliche Proteinfraktion enthält, die aus einem Nahrungsmittel gewonnen und zur Prüfung der menschlichen Nahrungsempfindlichkeit geeignet ist, mit den Schritten:
   Bereiten einer wässrigen Lösung der wasserlöslichen Proteinfraktion aus einer Nahrungsmittel/Wasser-Dispersion unter steriler Filterung;
   Einbringen der wässrigen Lösung der wasserlöslichen Fraktion in eine in einer Probenplatte ausgebildete Vertiefung; und
   Gefriertrocknen der wässrigen Lösung zur Ablagerung der Fraktion in der Vertiefung.

2. Verfahren nach Anspruch 1, **dadurch gekennzeichnet, daß** das Nahrungsmittel in dem 150- bis 1.500-fachen seines Eigengewichts in gereinigtem Wasser dispergiert ist.

3. Verfahren nach Anspruch 1 oder 2, **dadurch gekennzeichnet, daß** die wässrige Lösung der Fraktion in einem automatisierten Verfahren eingebracht wird.

4. Verfahren nach einem der vorhergehenden Ansprüche, **dadurch gekennzeichnet, daß** 20 bis 40 $\mu$l der wässrigen Lösung in die Vertiefung eingebracht werden.

5. Verfahren nach einem der vorhergehenden Ansprüche, **dadurch gekennzeichnet, daß** die in der Vertiefung eingebrachte wässrige Lösung vor dem Schritt des Gefriertrocknens gefroren wird.

6. Verfahren nach einem der vorhergehenden Ansprüche, **dadurch gekennzeichnet, daß** das Gefriertrocknen für eine Zeitdauer von 1 bis 10 Stunden ausgeführt wird.

7. Verfahren nach einem der vorhergehenden Ansprüche, **dadurch gekennzeichnet, daß** das Gefriertrocknen bei einer Temperatur von -40° bis -70° C durchgeführt wird.

8. Verfahren nach einem der vorhergehenden Ansprüche, **dadurch gekennzeichnet, daß** das Gefriertrocknen bei einem Druck von $1 \times 10^{-4}$ bis $1 \times 10^{-3}$ Millibar ausgeführt wird.

9. Platte zur Verwendung bei der Prüfung der menschlichen Empfindlichkeit gegenüber einer Mehrzahl unterschiedlicher Nahrungsmittel, mit einer Vielzahl von Vertiefungen, in denen jeweils eine wasserlösliche Proteinfraktion eingebracht ist, die aus einem unterschiedlichen Nahrungsmittel gewonnen und jeweils gemäß dem Verfahren nach einem der Ansprüche 1 bis 8 erhältlich ist.

10. Verfahren zum In-vitro-Prüfen der menschlichen Empfindlichkeit gegenüber verschiedenen Nahrungsmitteln, **gekennzeichnet durch** den Schritt der Überprüfung menschlicher Granulozyten, die zu der Vielzahl unterschiedlicher wasserlöslicher Proteinfraktionen hinzugefügt sind, die in den Vertiefungen einer Platte gemäß Anspruch 9 eingebracht sind.